(19) Europäisches Patentamt
European Patent Office
Office européen des brevets

(11) Veröffentlichungsnummer: **0 130 939**
**B1**

(12) # EUROPÄISCHE PATENTSCHRIFT

(45) Veröffentlichungstag der Patentschrift: **02.11.88**

(21) Anmeldenummer: **84810269.5**

(22) Anmeldetag: **30.05.84**

(51) Int. Cl.⁴: **A 01 N 25/32,** A 01 N 43/66, C 07 D 251/22, C 07 D 251/24 // (A01N43/66, 37:26, 37:24, 37:22, 37:20)

(54) **Verwendung von Triazin-Derivaten zum Schützen von Mais- und Sorghumpflanzen.**

(30) Priorität: **06.06.83 CH 3085/83**

(43) Veröffentlichungstag der Anmeldung: **09.01.85 Patentblatt 85/2**

(45) Bekanntmachung des Hinweises auf die Patenterteilung: **02.11.88 Patentblatt 88/44**

(84) Benannte Vertragsstaaten: **AT BE CH DE FR IT LI NL**

(56) Entgegenhaltungen:
**DE-B-1 174 321**
**DE-B-1 217 386**
**US-A-4 340 419**

**R. WEGLER: "Chemie der Pflanzenschutz- und Schädlingsbekämpfungsmittel", Band 8, 1982, Seiten 90-93, Springer-Verlag, Berlin, DE**

(73) Patentinhaber: **CIBA- GEIGY AG, Klybeckstrasse 141, CH- 4002 Basel (CH)**

(72) Erfinder: **Szczepanski, Henry, Dr., Bodenmatt, CH- 4323 Wallbach (CH)**
Erfinder: **Berrer, Dagmar, Dr., Steingrubenweg 92, CH- 4125 Riehen (CH)**

**EP 0 130 939 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft die Verwendung von Triazin-Derivaten zum Schützen von Mais- und Sorghumpflanzen gegen schädigende Wirkungen von herbiziden Chloracetaniliden und Mittel, welche solche Triazin-Derivate enthalten.

Beim Einsatz von herbiziden Chloracetaniliden können in Abhängigkeit von Faktoren wie beispielsweise Dosis und Applikationsart, Bodenbeschaffenheit und klimatischen Bedingungen, wie beispielsweise Belichtungsdauer, Temperatur und Niederschlagsmengen, Mais- und Sorghumpflanzen in gewissem Masse geschädigt werden. Eine Schädigung kann sowohl bei einer gezielten Herbizidbehandlung dieser Kulturpflanzen wie auch durch Abdriften oder Absickern der Herbizide während oder nach der Behandlung benachbarter Areale in den Bereich von Mais- und Sorghumbeständen oder durch Herbizidrückstände im Boden auftreten.

Aus der US Patentschrift 4 340 419 ist bekannt, dass Triazin-Derivate, wie z. B. das 2-Methyl-4,6-bis(trichlormethyl)-s-triazin und das 2-Äthylamino-4-chlor-6-[N-äthyl-N-(dichlor-methylcarbonyl)-amino]-s-triazin zum Schützen von Pflanzen gegen schädigende Wirkungen von herbiziden Thiolcarbamaten verwendet werden.

Ziel der vorliegenden Erfindung war neue Mittel zum Schutz von Kulturpflanzen gegen die schädigende Wirkung von Herbiziden anderer Art bereitzustellen.

Es wurde nun gefunden, dass sich überraschenderweise eine Gruppe von Triazin-Derivaten hervorragend dazu eignet, Mais- und Sorghumpflanzen gegen schädigende Wirkungen von herbiziden Chloracetaniliden zu schützen. Diese Triazin-Derivate werden daher im folgenden auch als "Gegenmittel", "Antidot" oder "Safener" bezeichnet.

Triazin-Derivate, welche zum Schützen von Mais- und Sorghumpflanzen gegen schädigende Wirkungen von herbiziden Chloracetaniliden geeignet sind, entsprechen der Formel I

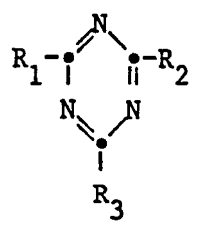

(I)

worin

$R_1$, $R_2$ und $R_3$ unabhängig voneinander $C_1$-$C_5$-Alkyl, welches unsubstituiert oder durch $C_3$-$C_6$-Cycloalkyl monosubstituiert oder durch Halogen ein- oder mehrfach substituiert ist, $C_3$-$C_6$-Cycloalkyl, welches unsubstituiert oder ein- oder mehrfach durch Halogen substituiert ist, bedeuten, oder einer der Substituenten $R_1$, $R_2$ und $R_3$ auch für Halogen oder $-NR_4R_5$ steht, worin $R_4$ Wasserstoff oder $C_1$-$C_5$-Alkyl und $R_5$ Wasserstoff, $C_1$-$C_5$-Alkyl, Monochloracetyl oder Dichloracetyl bedeuten.

$C_1$-$C_5$-Alkyl umfasst geradkettige und verzweigte Alkylreste, wie beispielsweise Methyl, Äthyl, n-Propyl, Isopropyl, iso.-Butyl, sek.-Butyl, tert.-Butyl, n-Pentyl und Isomere davon.

$C_3$-$C_6$-Cycloalkyl steht für Cyclopropyl, Cyclobutyl, Cyclopentyl und Cyclohexyl.

Unter Halogen als Substituent oder Teil eines Substituenten sind Fluor, Chlor, Brom und Jod, vorzugsweise Chlor, zu verstehen.

Halogenalkyl steht für einfach halogenierte bis perhalogenierte Alkylsubstituenten, wie beispielsweise $-CH_2Br$, $-CHF_2$, $-CHCl_2$, $-CH_2J$, $-CH_2CCl_3$, $-CH_2CH_2Cl$, $-CH_2CH_2CF_3$, $-CH_2CH_2CH_2Br$, $-CH_2CHClCH_2Cl$, $-CH_2CH_2CH_2CH_2Br$, $-CCl_2CCl_3$, $CF_3$ und vor allem $-CH_2Cl$, $-CH_2CH_2CH_2Cl$, $-CH_2CH_2CH_2CH_2Cl$ und $-CCl_3$.

Besonders geeignet zur erfindungsgemässen Verwendung sind Verbindungen der Formel I, in denen zwei der Substituenten $R_1$, $R_2$ und $R_3$ unabhängig voneinander $C_1$-$C_5$-Alkyl, welches unsubstituiert oder durch $C_3$-$C_6$-Cycloalkyl, insbesondere Cyclopropyl, monosubstituiert oder durch Halogen ein- oder mehrfach substituiert ist, $C_3$-$C_6$ Cycloalkyl, welches unsubstituiert oder ein- oder mehrfach durch Halogen substituiert ist, bedeuten, oder einer der Substituenten auch für Halogen steht, und der dritte Substituent für $C_3$-$C_6$-Cycloalkyl steht, insbesondere für Cyclopropyl.

Für die erfindungsgemässe Verwendung sehr gut geeignete Einzelverbindungen sind:
2-Methyl-4,6-bis(trichlormethyl)-s-triazin,
2-Chlormethyl-4,6-bis(trichlormethyl)-s-triazin,
2-Amino-4-chlormethyl-6-trichlormethyl-s-triazin,
2-(3-Chlor-n-propyl)-4-methyl-6-trichlormethyl-s-triazin,
2-(3-Chlor-n-propyl)-4-cyclopropyl-6-trichlormethyl-s-triazin,
2-(4-Chlor-n-butyl)-4,6-bis(trichlormethyl)-s-triazin,
2-Amino-4-(3-chlor-n-propyl)-6-trichlormethyl-s-triazin,
2-Cyclopropyl-4,6-bis(trichlormethyl)-s-triazin,
2-Amino-4-cyclopropyl-6-trichlormethyl-s-triazin,
2-(3-Chlor-n-propyl)-4,6-bis(trichlormethyl)-s-triazin,

2-Äthyl-4-(3-chlor-n-propyl)-6-trichlormethyl-s-triazin,
2-(3-Chlor-n-propyl)-4-n-propyl-6-trichlormethyl-s-triazin,
2-Amino-4-cyclopropyl-6-methyl-s-triazin,
2-(Chloracetyl)-amino-4-(3-chlor-n-propyl)-6-methyl-s-triazin
2-Amino-4,6-dimethyl-s-triazin,
2-(Dichloracetyl)-amino-4-(3-chlor-n-propyl)-6-methyl-s-triazin
2-Äthylamino-4-chlor-6-[N-äthyl-N-(dichloracetyl)-amino]-s-triazin
2-Methylamino-4-cyclopropyl-6-trichlormethyl-s-triazin
2-Äthylamino-4-cyclopropyl-6-trichlormethyl-s-triazin
2-n-Propylamino-4-cyclopropyl-6-trichlormethyl-s-triazin
2-Isopropylamino-4-cyclopropyl-6-trichlormethyl-s-triazin
2-Dimethylamino-4-cyclopropyl-6-trichlormethyl-s-triazin
2-Amino-4,6-bis(trichlormethyl)-s-triazin
2-Chlor-4,6-bis(trichlormethyl)-s-triazin
2-Äthylamino-4,6-bis(trichlormethyl)-s-triazin
2,4,6-Tris(trichlormethyl)-s-triazin
2-Methyl-4-methylamino-6-trichlormethyl-s-triazin
2-Amino-4-methyl-6-trichlormethyl-s-triazin
2-Isopropyl-4,6-bis(trichlormethyl)-s-triazin
2-Äthyl-4,6-bis(trichlormethyl)-s-triazin
2-Amino-4-dichlormethyl-6-methyl-s-triazin
2-Amino-4-cyclopropyl-6-dichlormethyl-s-triazin.

Als zu antagonisierende Substanzen kommen Chloracetanilide in Betracht, welche herbizide Wirkung aufweisen und der Formel II

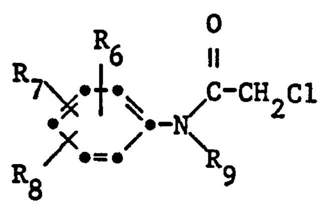

(II)

worin
$R_6$ Wasserstoff, $C_1$-$C_4$-Alkyl, Methoxy oder Chlor,
$R_7$ Wasserstoff oder $C_1$-$C_3$-Alkyl und
$R_8$ Wasserstoff, Methyl oder Äthyl bedeuten, mit der Massgabe, dass $R_6$, $R_7$ und $R_8$ gemeinsam nicht mehr als 6 Kohlenstoffatome aufweisen, und
$R_9$ für Äthyl, Isopropyl, 1-Methyl-2-methoxyäthyl, $-CH_2-R_{10}$, $-CH(CH_3)-R_{11}$, $-CH_2-CH(R_{12})-R_{13}$, $-CH(CH_3)-CH(R_{14})-R_{15}$, $C_3$-$C_4$-Alkinyl oder 5-Methyl-1,3,4-oxadiazol-2-yl steht, worin $R_{10}$ $C_1$-$C_4$-Alkoxy, $C_1$-$C_3$-Alkoxycarbonyl, Dimethylaminocarbonyl, 2-Propinylaminocarbonyl, Benzoyl, 4-Chlorbenzoyl, Cyan, 2-Furanyl, 2-Tetrahydrofuranyl, 1,3-Dioxolan-2-yl, 1,3-Dioxan-2-yl, 1H-Pyrazol-1-yl, 3,5-Dimethyl-1H-pyrazol-1-yl, 1H-1,2,4-Triazol-1-yl, 1-Methyl-5-methylthio-1,3,4-triazol-2-yl oder $-P(O)(OR_{16})(OR_{17})$, worin $R_{16}$ und $R_{17}$ unabhängig voneinander für Methyl oder Äthyl stehen, $R_{11}$ Methoxycarbonyl oder 1,3-Dioxolan-2-yl, $R_{12}$ Wasserstoff und $R_{13}$ $C_1$-$C_4$-Alkoxy oder Allyloxy oder $R_{12}$ Methyl und $R_{13}$ $C_1$-$C_2$-Alkoxy oder $R_{12}$ und $R_{13}$ Äthoxy und $R_{14}$ Wasserstoff und $R_{15}$ $C_1$-$C_2$-Alkoxy oder $R_{14}$ Methyl und $R_{15}$ Methoxy bedeuten, entsprechen.

Als zu antagonisierende, herbizid wirksame Chloracetanilide kommen insbesondere die folgenden in Betracht:

H-1. N-Chloracetyl-N-(2-methoxy-1-methyläthyl)-2,6-diäthylanilin,
H-2. N-Chloracetyl-N-(2-methoxy-1-methyläthyl)-2-äthyl-6-methylanilin,
H-3. N-(2-Äthoxyäthyl)-N-chloracetyl-2,6-diäthylanilin,
H-4. N-Chloracetyl-N-(2-n-propoxyäthyl)-2-äthyl-6-methylanilin,
H-5. N-Chloracetyl-N-(2-n-propoxyäthyl)-2,6-diäthylanilin,
H-6. N-Chloracetyl-N-(2-isopropoxyäthyl)-2-äthyl-6-methylanilin,
H-7. N-Äthoxycarbonylmethyl-N-chloracetyl-2,6-dimethylanilin,
H-8. N-Äthoxycarbonylmethyl-N-chloracetyl-2,6-diäthylanilin,
H-9. N-Chloracetyl-N-methoxycarbonylmethyl-2,6-dimethylanilin,
H-10. N-Chloracetyl-N-(2,2-diäthoxyäthyl)-2,6-dimethylanilin,
H-11. N-Chloracetyl-N-(2-methoxy-1-methyläthyl)-2,3-dimethylanilin,
H-12. N-(2-Äthoxyäthyl)-N-chloracetyl-2-methylanilin,
H-13. N-Chloracetyl-N-(2-methoxyäthyl)-2-methylanilin,
H-14. N-Chloracetyl-N-(2-methoxy-2-methyläthyl)-2,6-dimethylanilin,
H-15. N-(2-Äthoxy-2-methyläthyl)-N-chloracetyl-2-äthyl-6-methylanilin,

H-16. N-Chloracetyl-N-(1-äthyl-2-methoxyäthyl)-2,6-dimethylanilin,
H-17. N-Chloracetyl-N-(2-methoxyäthyl)-2-methoxy-6-methylanilin,
H-18. N-n-Butoxymethyl-N-chloracetyl-2-tert.-butylanilin,
H-19. N-Chloracetyl-N-(2-äthoxyäthyl-1-methyläthyl)-2,6-dimethylanilin,
H-20. N-Chloracetyl-N-(2-methoxyäthyl)-2-chlor-6-methylanilin,
H-21. N-(2-Äthoxyäthyl)-N-chloracetyl-2-chlor-6-methylanilin,
H-22. N-(2-Äthoxyäthyl)-N-chloracetyl-2,3,6-trimethylanilin,
H-23. N-Chloracetyl-1-(2-methoxyäthyl)-2,3,6-trimethylanilin,
H-24. N-Chloracetyl-N-cyanomethyl-2,6-dimethylanilin,
H-25. N-But-3-in-1-yl-N-chloracetylanilin,
H-26. N-Chloracetyl-N-propargyl-2-äthyl-6-methylanilin,
H-27. N-Chloracetyl-N-(1,3-dioxolan-2-ylmethyl)-2,6-dimethylanilin,
H-28. N-Chloracetyl-N-(1,3-dioxolan-2-ylmethyl)-2-äthyl-6-methylanilin,
H-29. N-Chloracetyl-N-(1,3-dioxan-2-ylmethyl)-2-äthyl-6-methylanilin,
H-30. N-Chloracetyl-N-(2-furanylmethyl)-2,6-dimethylanilin,
H-31. N-Chloracetyl-N-(2-furanylmethyl)-2-äthyl-6-methylanilin,
H-32. N-Chloracetyl-N-(2-tetrahydrofuranylmethyl)-2,6-dimethylanilin,
H-33. N-Chloracetyl-N-(N-propargylcarbamoylmethyl)-2,6-dimethylanilin,
H-34. N-Äthoxymethyl-N-chloracetyl-2-äthyl-6-methylanilin,
H-35. N-Chloracetyl-N-methoxymethyl-2,6-diäthylanilin,
H-36. N-Chloracetyl-N-(2-methoxyäthyl)-2,6-dimethylanilin,
H-37. N-(2-Allyloxyäthyl)-N-chloracetyl-2,6-dimethylanilin,
H-38. N-Chloracetyl-N-(2-n-propoxyäthyl)-2,6-dimethylanilin,
H-39. N-Chloracetyl-N-(2-isopropoxyäthyl)-2,6-dimethylanilin,
H-40. N-Chloracetyl-N-(2-methoxyäthyl)-2-äthyl-6-methylanilin,
H-41. N-Chloracetyl-N-(methoxyäthyl)-2,6-diäthylanilin,
H-42. N-(2-Äthoxyäthyl)-N-chloracetyl-2-äthyl-6-methylanilin,
H-43. N-Chloracetyl-N-(2-methoxy-1-methyläthyl)-2-methylanilin,
H-44. N-Chloracetyl-N-(2-methoxy-1-methyläthyl)-2,6-dimethylanilin,
H-45. N-Chloracetyl-N-(N,N-dimethylcarbamoylmethyl)-2,6-dimethylanilin,
H-46. N-(n-Butoxymethyl)-N-chloracetyl-2,6-diäthylanilin,
H-47. N-(2-n-Butoxyäthyl)-N-chloracetyl-2,6-diäthylanilin,
H-48. N-Chloracetyl-N-(2-methoxy-1,2-dimethyläthyl)-2,6-dimethylanilin,
H-49. N-Chloracetyl-N-isopropyl-2,3-dimethylanilin,
H-50. N-Chloracetyl-N-isopropyl-2-chloranilin,
H-51. N-Chloracetyl-N-(1H-pyrazol-1-ylmethyl)-2,6-dimethylanilin,
H-52. N-Chloracetyl-N-(1H-pyrazol-1-ylmethyl)-2-äthyl-6-methylanilin,
H-53. N-Chloracetyl-N-(1H-1,2,4-triazol-1-ylmethyl)-2,6-dimethylanilin,
H-54. N-Chloracetyl-N-(1H-1,2,4-triazol-1-ylmethyl)-2,6-diäthylanilin,
H-55. N-Benzoylmethyl-N-chloracetyl-2,6-dimethylanilin,
H-56. N-Benzoylmethyl-N-chloracetyl-2-äthyl-6-methylanilin,
H-57. N-Chloracetyl-N-(5-methyl-1,3,4-oxadiazol-2-yl)-2,6-diäthylanilin,
H-58. N-Chloracetyl-N-(5-methyl-1,3,4-oxadiazol-2-yl)-2-äthyl-6-methylanilin,
H-59. N-Chloracetyl-N-(5-methyl-1,3,4-oxadiazol-2-yl)-2-tert.-butylanilin,
H-60. N-Chloracetyl-N-(4-chlorbenzoylmethyl)-2,6-dimethylanilin und
H-61. N-Chloracetyl-N-(3-methylthio-4-methyl-1,2,4-triazol-5-yl-methyl)-2,6-diäthylanilin, insbesondere die Chloracetanilide Nr. H-2, H-5, H-35 und H-44.

Durch Einsatz von Verbindungen der Formel I können Mais- und Sorghumpflanzen auch gegen Halogenacetanilide geschützt werden, welche in R. Wegler, Chemie der Pflanzenschutz- und Schädlingsbekämpfungsmittel, Springer-Verlag, Heidelberg, New York, 1982, Seiten 90-93 und 322-327, beschrieben sind.

Verfahren zur Herstellung von Verbindungen der Formeln II und III sind bekannt oder die Herstellung dieser Verbindungen kann analog bekannten Methoden erfolgen.

Ein geeignetes Verfahren zum Schützen von Mais- und Sorghumpflanzen gegen schädigende Wirkungen von herbiziden Chloracetaniliden der Formel II unter Verwendung von Verbindungen der Formel I besteht darin, dass man diese Pflanzen, Teile dieser Pflanzen oder die Anbauflächen für diese Pflanzen gleichzeitig mit einem Herbizid der Formel II oder vor oder nach der Anwendung eines Herbizids der Formel II mit einer antagonistisch wirksamen Menge einer Verbindung der Formel I oder einem Mittel, welches eine solche Verbindung enthält, behandelt.

Die Erfindung betrifft auch ein Verfahren zur selektiven Bekämpfung von Unkräutern in Mais- und Sorghumkulturen, wobei die Mais- oder Sorghumbestände, das Saatgut dieser Pflanzen oder Anbauflächen für diese Pflanzen gleichzeitig mit einem Herbizid der Formel II oder vor oder nach der Anwendung eines Herbizids

der Formel II mit einer antagonistisch wirksamen Menge einer Verbindung der Formel I oder einem Mittel, welches neben einer herbizid wirksamen Menge eines Chloracetanilids der Formel II eine die schädigende Wirkung des Herbizids II antagonisierende Menge einer Verbindug der Formel I enthält, behandelt werden. Mittel, welche eine solche Herbizid/Antidot-Kombination enthalten, bilden ebenfalls einen Bestandteil der vorliegenden Erfindung.

Bei den zu bekämpfenden Unkräutern kann es sich sowohl um monocotyle wie um dicotyle Unkräuter handeln.

Als Anbauflächen für Mais- oder Sorghumkulturen gelten die bereits mit den Kulturpflanzen bewachsenen oder die mit Saatgut dieser Kulturpflanzen beschickten Bodenareale, wie auch die zur Bebauung mit diesen Kulturpflanzen bestimmten Böden.

Die zu applizierende Aufwandmenge Antidot im Verhältnis zum Herbizid richtet sich weitgehend nach der Anwendungsart. Bei einer Feldbehandlung, welche entweder unter Verwendung einer Tankmischung mit einer Kombination von Antidot und Herbizid oder durch getrennte Applikation von Antidot und Herbizid erfolgt, liegt in der Regel ein Verhältnis von Antidot zu Herbizid von 1 : 100 bis 10 : 1, bevorzugt 1 : 5 bis 8 : 1, und insbesondere 1 : 1, vor. Dagegen werden bei der Samenbeizung weit geringere Mengen Antidot im Verhältnis zur Aufwandmenge an Herbizid/ha Anbaufläche benötigt. In der Regel werden bei der Samenbeizung 0,1 bis 10 g Antidot/kg Samen, bevorzugt 1 bis 2 g, appliziert. Wird das Gegenmittel kurz vor der Aussaat unter Samenquellung appliziert, so werden zweckmässigerweise Antidot-Lösungen verwendet, welche den Wirkstoff in einer Konzentration von 1 bis 10'000 ppm, bevorzugt 100 bis 1'000 ppm, enthalten.

Die Verbindungen der Formel I können ohne oder zusammen mit inerten Zusatzstoffen gleichzeitig mit einem Herbizid der Formel II oder vor oder nach der Anwendung eines Herbizids der Formel II zur Anwendung gelangen.

Die vorliegende Anmeldung betrifft daher auch Mittel, welche Verbindungen der Formel I, inerte Zusatzstoffe und zu antagonisierende Herbizide enthalten.

Zur Applikation werden die Verbindungen der Formel I gleichzeitig mit einem Herbizid der Formel II oder vor oder nach der Anwendung eines Herbizids der Formel II oder Kombinationen von Verbindungen der Formel I mit zu antagonisierenden Herbiziden der Formel II zweckmässigerweise zusammen mit den in der Formulierungstechnik üblichen Hilfsmitteln eingesetzt und werden daher z. B. zu Emulsionskonzentraten, streichfähigen Pasten, direkt versprühbaren oder verdünnbaren Lösungen verdünnten Emulsionen Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt.

Die Formulierungen, d. h., die den Wirkstoff der Formel I oder eine Kombination von Wirkstoff der Formel I mit zu antagonisierendem Herbizid der Formel II und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht-sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffs der Formel I und gegebenenfalls auch dem zu antagonisierenden Herbizid nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z. B. die Na- oder K-Salze der Öl oder Stearinsäure, oder von natürlichen Fettsäuregemischen, die z. B. aus Kokosnuss- oder Talgöl gewonnen werden können, genannt. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte

Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxyd-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Äthylenoxyd-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 100 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyldi(2-chloräthyl)-äthylammoniumbromid.

Die in der Formulierungstechnik gebräuchlichen Tenside sind u. a. in folgenden Publikationen beschrieben:

"Mc Cutcheon's Detergents and Emulsifiers Annual" MC Publishing Corp., Ridgewood New Jersey, 1981

Stache, H., "Tensid-Taschenbuch", Carl Hanser Verlag, München/Wien, 1981

Die agrochemischen Zubereitungen enthalten in der Regel 0,1 bis 99 Gewichtsprozent, insbesondere 0,1 bis 95 Gew.-%, Wirkstoff der Formel I oder Wirkstoffgemisch Antidot/Herbizid, 1 bis 99,9 Gew.-%, insbesondere 5 bis 99,8 Gew.-%, eines festen oder flüssigen Zusatzstoffes und 0 bis 25 Gew.-%, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Für die Verwendung von Verbindungen der Formel I gleichzeitig mit einem Herbizid der Formel II oder vor oder nach der Anwendung eines Herbizids der Formel II oder sie enthaltender Mittel zum Schützen von Kulturpflanzen gegen schädigende Wirkungen von Chloracetaniliden der Formel II kommen verschiedene Methoden und Techniken in Betracht, wie beispielsweise die folgenden:

1) <u>Samenbeizung</u>

a) Beizung der Samen mit einem als Spritzpulver formulierten Wirkstoff der Formel I durch Schütteln in einem Gefäss bis zur gleichmässigen Verteilung auf der Samenoberfläche (Trockenbeizung). Man verwendet dabei etwa 10 bis 500 g Wirkstoff der Formel I (40 g bis 2 kg Spritzpulver) pro 100 kg Saatgut.

b) Beizung der Samen mit einem Emulsionskonzentrat des Wirkstoffs der Formel I nach der Methode a) (Nassbeizung).

c) Beizung durch Tauchen des Saatguts in eine Brühe mit 50 - 3200 ppm Wirkstoff der Formel I während 1 bis 72 Stunden und gegebenenfalls nachfolgendes Trocknen der Samen (Tauchbeizung).

Die Beizung des Saatguts oder die Behandlung des angekeimten Sämlings sind naturgemäss die bevorzugten Methoden der Applikation, weil die Wirkstoffbehandlung vollständig auf die Zielkultur gerichtet ist. Man verwendet in der Regel 10 g bis 500 g Antidot, vorzugsweise 50 bis 250 g Antidot, pro 100 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

2) <u>Applikation aus Tankmischung</u>

Eine flüssige Aufarbeitung eines Gemisches von Antidot und Herbizid (gegenseitiges Mengenverhältnis vorzugsweise zwischen 10 : 1 und 1 : 10) wird verwendet, wobei die Aufwandmenge an Herbizid 0,1 bis 10 kg pro Hektar beträgt. Solche Tankmischung wird vorzugsweise vor oder unmittelbar nach Aussaat appliziert oder 5 bis 10 cm tief in den noch nicht gesäten Boden eingearbeitet.

3) <u>Applikation in der Saatfurche</u>

Das Antidot wird als Emulsionskonzentrat, Spritzpulver oder als Granulat in die offene besäte Saatfurche eingebracht und hierauf wird nach dem Decken der Saatfurche in normaler Weise das Herbizid im Vorauflaufverfahren appliziert.

4) Kontrollierte Wirkstoffabgabe

Der Wirkstoff der Formel I wird in Lösung auf mineralische Granulatträger oder polymerisierte Granulate (Harnstoff/Formaldehyd) aufgezogen und trocknen gelassen. Gegebenenfalls kann ein Überzug aufgebracht werden (Umhüllungsgranulate), der es erlaubt, den Wirkstoff über einen bestimmten Zeitraum dosiert abzugeben.

Die Triazin-Derivate der Formel I sind teils neu, teils bekannt. So sind u. a. die Verbindungen 2-Methyl-4,6-bis(trichlormethyl)-s-triazin, 2-Chlormethyl-4,6-bis(trichlormethyl)-s-triazin und 2-Äthylamino-4-chlor-6-[N-äthyl-N-(dichlormethylcarbonyl)-amino]-s-triazin in der US-PS-4 340 419 beschrieben.

Neue Triazin-Derivate sind beispielsweise Verbindungen der unter die allgemeine Formel I fallenden Formel Ia

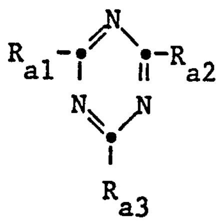

(Ia)

worin

einer der Substituenten $R_{a1}$, $R_{a2}$ und $R_{a3}$ für $C_1$-$C_5$-Alkyl, welches unsubstituiert oder durch $C_3$-$C_6$-Cycloalkyl monosubstituiert oder ein- oder mehrfach durch Halogen substituiert ist, oder für $C_3$-$C_6$-Cycloalkyl, welches unsubstituiert oder ein- oder mehrfach durch Halogen substituiert ist, steht,

ein weiterer der Substituenten $R_{a1}$, $R_{a2}$ und $R_{a3}$ n-Pentyl oder ein Isomeres davon, $C_1$-$C_5$-Alkyl, welches durch $C_3$-$C_6$-Cycloalkyl monosubstituiert ist, $C_4$-$C_5$-Alkyl, welches ein- oder mehrfach durch Halogen substituiert ist, $C_3$-$C_6$-Cycloalkyl, welches unsubstituiert oder ein- oder mehrfach durch Halogen substituiert ist, oder ein Jodatom bedeutet, und

der dritte der Substituenten $R_{a1}$, $R_{a2}$ und $R_{a3}$ für $C_1$-$C_5$-Alkyl, welches unsubstituiert oder durch $C_3$-$C_6$-Cycloalkyl monosubstituiert oder ein- oder mehrfach durch Fluor, Brom oder Jod substituiert ist, $C_2$-$C_5$-Alkyl, welches ein- oder mehrfach durch Chlor substituiert ist, Chlormethyl, Dichlormethyl, $C_3$-$C_6$-Cycloalkyl, welches unsubstituiert oder ein- oder mehrfach durch Halogen substituiert ist, oder für $-NR_{a4}R_{a5}$, worin $R_{a4}$ Wasserstoff oder $C_1$-$C_5$-Alkyl und $R_{a5}$ n-Pentyl oder ein Isomeres davon, Monochloracetyl oder Dichloracetyl bedeutet, steht.

Von diesen Verbindungen sind vor allem die unter die Formel Ia fallenden Verbindungen der Formel Ib

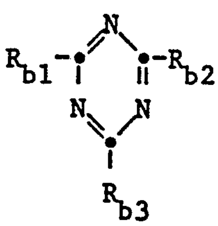

(Ib)

worin

einer der Substituenten $R_{b1}$, $R_{b2}$ und $R_{b3}$ für $C_1$-$C_5$-Alkyl, welches unsubstituiert oder durch $C_3$-$C_6$-Cycloalkyl monosubstituiert oder ein- oder mehrfach durch Halogen substituiert ist, oder für $C_3$-$C_6$-Cycloalkyl, welches unsubstituiert oder ein- oder mehrfach durch Halogen substituiert ist, steht,

ein weiterer der Substituenten $R_{b1}$, $R_{b2}$ und $R_{b3}$ n-Pentyl oder ein Isomeres davon, $C_1$-$C_5$-Alkyl, welches durch $C_3$-$C_6$-Cycloalkyl monosubstituiert ist, $C_4$-$C_5$-Alkyl, welches ein- oder mehrfach durch Halogen substituiert ist, $C_3$-$C_6$-Cycloalkyl, welches unsubstituiert oder ein- oder mehrfach durch Halogen substituiert ist, oder ein Jodatom bedeutet, und

der dritte der Substituenten $R_{b1}$, $R_{b2}$ und $R_{b3}$ für $C_1$-$C_5$-Alkyl, welches unsubstituiert oder durch $C_3$-$C_6$-Cycloalkyl monosubstituiert oder ein- oder mehrfach durch Fluor, Brom oder Jod substituiert ist, $C_2$-$C_5$-Alkyl, welches ein- oder mehrfach durch Chlor substituiert ist, Chlormethyl, Dichlormethyl, $C_3$-$C_6$-Cycloalkyl, welches unsubstituiert oder ein- oder mehrfach durch Halogen substituiert ist, oder für $-NR_{b4}R_{b5}$, worin $R_{b4}$ Wasserstoff oder $C_1$-$C_5$-Alkyl und $R_{b5}$ n-Pentyl oder ein Isomeres davon, Monochloracetyl oder Dichloracetyl bedeutet, steht,

mit der Massgabe, dass mindestens einer der Substituenten $R_{b1}$, $R_{b2}$ und $R_{b3}$ für $C_1$-$C_5$-Alkyl, welches durch

$C_3$-$C_6$-Cycloalkyl monosubstituiert ist, für Cycloalkyl, welches unsubstituiert oder ein- oder mehrfach durch Halogen substituiert ist, ein Jodatom oder $-NR_{b4}R_{b5}$, worin $R_{b4}$ Wasserstoff oder $C_1$-$C_5$-Alkyl und $R_{b5}$ Monochloracetyl bedeuten oder $R_{b4}$ Methyl, n-Propyl, Isopropyl, n-Butyl, sec.-Butyl, tert.-Butyl oder Isobutyl und $R_{b5}$ Dichloracetyl bedeutet, steht,
erwähnenswert.

Von diesen Verbindungen sind insbesondere die unter die Formel Ib fallenden Verbindungen der Formel Ic

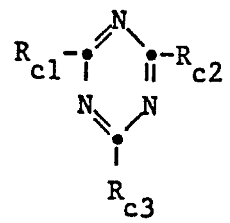

(Ic)

worin
einer der Substituenten $R_{c1}$, $R_{c2}$ und $R_{c3}$ für $C_1$-$C_5$-Alkyl, welches unsubstituiert oder durch $C_3$-$C_6$-Cycloalkyl monosubstituiert oder ein- oder mehrfach durch Halogen substituiert ist, oder für $C_3$-$C_6$-Cycloalkyl, welches unsubstituiert oder ein- oder mehrfach durch Halogen substituiert ist, steht,
ein weiterer der Substituenten $R_{c1}$, $R_{c2}$ und $R_{c3}$ n-Pentyl oder ein Isomeres davon, $C_1$-$C_5$-Alkyl, welches durch $C_3$-$C_6$-Cycloalkyl monosubstituiert ist, $C_4$-$C_5$-Alkyl, welches ein- oder mehrfach durch Halogen substituiert ist, $C_3$-$C_6$-Cycloalkyl, welches unsubstituiert oder ein- oder mehrfach durch Halogen substituiert ist, oder ein Jodatom bedeutet, und
der dritte der Substituenten $R_{c1}$, $R_{c2}$ und $R_{c3}$ für $C_1$-$C_5$-Alkyl, welches unsubstituiert oder durch $C_3$-$C_6$-Cycloalkyl monosubstituiert oder ein- oder mehrfach durch Fluor, Brom oder Jod substituiert ist, $C_2$-$C_5$-Alkyl, welches ein- oder mehrfach durch Chlor substituiert ist, Chlormethyl, Dichlormethyl oder für $C_3$-$C_6$-Cycloalkyl, welches unsubstituiert oder ein- oder mehrfach durch Halogen substituiert ist,
mit der Massgabe, dass mindestens einer der Substituenten $R_{c1}$, $R_{c2}$ und $R_{c3}$ für Cycloalkyl, welches unsubstituiert oder ein- oder mehrfach durch Halogen substituiert ist, steht,
zu beachten.

Von den Verbindungen der Formel Ic sind insbesondere diejenigen von Interesse, bei denen als Cycloalkylgruppe ein unsubstituiertes Cyclopropyl vorliegt.

Die Verbindungen der Formel I lassen sich nach an sich bekannten Methoden herstellen, beispielsweise durch Kondensation entsprechend substituierter Nitrile oder durch Austausch von Substituenten am Triazinring. So lassen sich beispielsweise durch Trichlormethyl oder Methoxy substituierte Triazine durch Behandlung mit Ammoniak in die entsprechenden, durch Amino substituierten Triazine überführen. Verbindungen, in denen $R_5$, $R_{a5}$ oder $R_{b5}$ für Mono- oder Dichloracetyl steht, können hergestellt werden, indem man ein entsprechendes, durch Amino oder Alkylamino substituiertes Triazin-Derivat mit Mono- oder Dichloracetylchlorid umsetzt.

**Beispiel 1:** 2-(3-Chlor-n-propyl)-4-methyl-6-(trichlormethyl)-1,3,5-triazin (Verbindung Nr. 7).

Eine Lösung von 5 g 4-Chlor-n-butyronitril in 15 g Trichloracetonitril wird bei Raumtemperatur mit Salzsäure gesättigt und das Reaktionsgemisch 14 Stunden bei Raumtemperatur stehengelassen. Der ausgefallene Niederschlag wird abgenutscht und zusammen mit 8 g Acetimidoäthylester-hydrochlorid in 50 ml Acetonitril suspendiert. Zu dieser Suspension lässt man 10 g Triäthylamin zutropfen. Nach Abklingen der exothermen Reaktion wird das Gemisch 1 Stunde bei Raumtemperatur gerührt. Anschliessend wird vom ausgefallenen Triäthylammonium-hydrochlorid abfiltriert und eingedampft. Das Rohprodukt wird an Siliziumoxyd chromatographiert (Eluierung mit Äther/Hexan 1 : 10) und man erhält 4,5 g 2-(3-Chlor-n-propyl)-4-methyl-6-(trichlormethyl)-1,3,5-triazin als schwach gelbes Öl, $n_D^{22} = 1,5290$.

**Beispiel 2:** 2-Cyclopropyl-4,6-bis(trichlormethyl)-1,3,5-triazin (Verbindung Nr. 18)

Man kühlt 760 g Trichloracetonitril auf -15°C ab und bläst Chlorwasserstoff-Gas durch die Lösung bis zur Sättigung. Dann gibt man langsam unter Kühlung und Chlorwasserstoffgas-Zugabe 230 g Cyclopropylnitril zu, so dass die Temperatur nicht über -10°C steigt.

Dann wird das Kühlbad weggenommen und das Reaktionsgemisch bei Raumtemperatur gerührt, bis sich die Temperatur auf 15°C erhöht. Ab 10°C setzt mit mässig exothermer Reaktion Chlorwasserstoff-Entwicklung ein, welche nach 20 Stunden Rühren bei 15 - 20°C schliesslich beendet ist. Man gibt nun je 1,5 l Äther und Hexan zum Reaktionsgemisch, rührt das Ganze gut durch und filtriert. Das Filtrat wird eingeengt, der Rückstand in 700

ml Hexan aufgekocht, nochmals filtriert und das Filtrat erneut eingedampft. Der Rückstand wird aus Methanol umkristallisiert. Man erhält 350 g des obigen Triazins vom Schmelzpunkt 100 - 102° C.

**Beispiel 3:** 2-Amino-4-cyclopropyl-6-trichlormethyl-1,3 5-triazin (Verbindung Nr. 21)

Man löst 71,2 g 2-Cyclopropyl-4,6-bis-trichlormethyl-1,3,5-triazin in 70 ml Tetrahydrofuran und gibt bei Raumtemperatur unter Rühren 300 ml konzentrierte wässrige Ammoniaklösung zu. Man rührt während 30 Minuten, verdünnt dann mit Wasser und extrahiert die entstandene Emulsion mit Äther. Die Ätherphasen werden gesammelt, getrocknet, filtriert und eingedampft. Man erhält 48 g kristallines Titelprodukt. Schmelzpunkt 111-114° C.

**Beispiel 4:** 2-Amino-4-(3-chlor-n-propyl)-6-trichlormethyl-1,3,5-triazin (Verbindung Nr. 12)

Man löst 102,3 g 2-(3-Chlor-n-propyl)-4,6-bis-(trichlormethyl)-1,3,5-triazin in 100 ml Tetrahydrofuran und versetzt diese Lösung unter Rühren bei Raumtemperatur mit 400 ml konzentrierter wässriger Ammoniaklösung. Nach 30 Minuten gibt man 500 ml Wasser dazu und extrahiert das Reaktionsgemisch zweimal mit je 100 ml Äther. Die Ätherphasen werden über Magnesiumsulfat getrocknet, filtriert und eingedampft. Es verbleiben 68,3 g Titelprodukt als hellbraunes Öl.

**Beispiel 5:** 2-(3-Chlor-n-propyl)-4,6-bis(trichlormethyl)-1,3,5-triazin (Verbindung Nr. 4)

Man bläst bei einer Temperatur von -20° C gasförmigen Chlorwasserstoff durch eine Lösung von 103 g 4-Chlorbutyronitril in 298 g Trichloracetonitril, bis zur Sättigung. Dann wird langsam unter Rühren auf Raumtemperatur erwärmt, wobei nur eine relativ geringe Gasentwicklung stattfindet und ein kristalliner Niederschlag ausfällt. Man gibt noch einen Liter Toluol zu und rührt das Reaktionsgemisch bei 85° C bis sich kein HCl-Gas mehr entwickelt. Man hört dann mit Rühren auf, lässt erkalten und dekantiert die klare Lösung vom Schlamm, der sich gesetzt hat. Die Lösung wird eingedampft und das verbleibende Öl im Hochvakuum destilliert. Man ehält so 294 g Titelverbindung. Siedepunkt 150 - 160° C/0,2 mbar. Brechungsindex $n_D^{27}$: 1,5498.

Analog einer der vorstehend beschriebenen Methoden lassen sich auch die folgenden, in der Tabelle 1 zusammen mit den Verbindungen der vorstehenden Beispiele aufgeführten Verbindungen der Formel I herstellen:

**Tabelle 1**

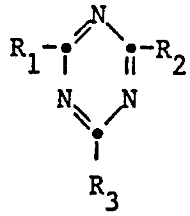

| Verbindung Nr. | $R_1$ | $R_2$ | $R_3$ | physikalische Konstante |
|---|---|---|---|---|
| 1 | $CCl_3$ | $CCl_3$ | $CH_3$ | Smp. 93 - 94°C |
| 2 | $CCl_3$ | $NHC_2H_5$ | Cyclopropyl | Smp. 47 - 49°C |
| 3 | $CH_3$ | $NH-CO-CHCl_2$ | $CH_2CH_2CH_2Cl$ | |
| 4 | $CCl_3$ | $CCl_3$ | $CH_2CH_2CH_2Cl$ | $n_D^{27} = 1{,}5498$ |
| 5 | $CCl_3$ | $NHCH_3$ | Cyclopropyl | Smp. 100 - 102°C |
| 6 | Cl | $N(C_2H_5)-CO-CHCl_2$ | $NHC_2H_5$ | Smp. 99 - 101°C |
| 7 | $CCl_3$ | $CH_3$ | $CH_2CH_2CH_2Cl$ | $n_D^{22} = 1{,}5290$ |
| 8 | $CCl_3$ | $NHnC_3H_7$ | Cyclopropyl | Smp. 66 - 68°C |
| 9 | $CCl_3$ | $CCl_3$ | $CH_2Cl$ | Smp. 59 - 61°C |
| 10 | $CH_3$ | $NH-CO-CH_2Cl$ | $CH_2CH_2CH_2Cl$ | |
| 11 | $CCl_3$ | $C_2H_5$ | $CH_2CH_2CH_2Cl$ | |
| 12 | $CCl_3$ | $NH_2$ | $CH_2CH_2CH_2Cl$ | Öl |
| 13 | $CCl_3$ | $CCl_3$ | $CH_2CH_2CH_2CH_2Cl$ | Öl |
| 14 | $CCl_3$ | $N(CH_3)_2$ | Cyclopropyl | Smp. 59 - 61°C |
| 15 | $CH_3$ | $NH_2$ | $CH_3$ | |
| 16 | $CCl_3$ | Cyclopropyl | $CH_2CH_2CH_2Cl$ | $n_D^{22} = 1{,}5325$ |
| 17 | $CH_3$ | $NH_2$ | Cyclopropyl | |
| 18 | $CCl_3$ | $CCl_3$ | Cyclopropyl | Smp. 100 - 102°C |
| 19 | $CCl_3$ | $NHisoC_3H_7$ | Cyclopropyl | Öl |
| 20 | $CCl_3$ | $nC_3H_7$ | $CH_2CH_2CH_2Cl$ | |
| 21 | $CCl_3$ | $NH_2$ | Cyclopropyl | Smp. 111 - 114°C |
| 22 | $CCl_3$ | $NH_2$ | $CH_2Cl$ | Smp. 109 - 113°C |
| 23 | $CCl_3$ | $NH_2$ | $CCl_3$ | Smp. 160 - 161°C |
| 24 | $CCl_3$ | $CCl_3$ | Cl | Smp. 54 - 57°C |
| 25 | $CCl_3$ | $NHC_2H_5$ | $CCl_3$ | Smp. 77 - 79°C |
| 26 | $CCl_3$ | $CCl_3$ | $CCl_3$ | fest |
| 27 | $CCl_3$ | $NHCH_3$ | $CH_3$ | Smp. 125 - 131°C |
| 28 | $CCl_3$ | $NH_2$ | $CH_3$ | Smp. 154 - 157°C |
| 29 | $CCl_3$ | $CCl_3$ | $isoC_3H_7$ | Öl |
| 30 | $CCl_3$ | $CCl_3$ | $C_2H_5$ | Smp. 37 - 39°C |
| 31 | $CHCl_2$ | $NH_2$ | $CH_3$ | Smp. 114 - 115°C |
| 32 | $CHCl_2$ | $NH_2$ | Cyclopropyl | Smp. 114 - 116°C |
| 33 | $CHCl_2$ | $NH-CO-CHCl_2$ | $CH_3$ | |
| 34 | $CHCl_2$ | $NH-CO-CH_2Cl$ | Cyclopropyl | |
| 35 | $CHCl_2$ | $NH-CO-CH_2Cl$ | $CH_3$ | |
| 36 | $CHCl_2$ | $NH-CO-CHCl_2$ | Cyclopropyl | |

**Formulierungsbeispiele für flüssige Wirkstoffe der Formel I** (% = Gewichtsprozent)

| 6. Emulsions-Konzentrate | a) | b) | c) |
|---|---|---|---|
| Wirkstoff aus Tabelle 1 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 7. Lösungen | a) | b) | c) | d) |
|---|---|---|---|---|
| Wirkstoff aus Tabelle 1 | 80 % | 10 % | 5 % | 95 % |
| Äthylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160 - 190°) | - | - | 94 % | - |

Die Lösungen sind zur Anwendung in Form kleinster Tropfen geeignet.

| 8. Granulate | a) | b) |
|---|---|---|
| Wirkstoff aus Tabelle 1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 9. Stäubemittel | a) | b) |
|---|---|---|
| Wirkstoff aus Tabelle 1 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

**Formulierungsbeispiele für feste Wirkstoffe der Formel I** (% = Gewichtsprozent)

| 10. Spritzpulver | a) | b) | c) |
|---|---|---|---|
| Wirkstoff aus Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7 - Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 11. Emulsions-Konzentrate | |
|---|---|
| Wirkstoff aus Tabelle 1 | 10 % |
| Octylphenolpolyäthylenglykoläther (4 - 5 Mol AeO) | 3 % |
| Ca-Dodecylbenzolsulfonat | 3 % |
| Ricinusölpolyglykoläther (35 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 12. Stäubemittel | a) | b) |
|---|---|---|
| Wirkstoff aus Tabelle 1 | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

| 13. Extruder Granulate | |
|---|---|
| Wirkstoff aus Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

14. Umhüllungs-Granulate

| | |
|---|---|
| Wirkstoff aus Tabelle 1 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

15. Suspensions-Konzentrate

| | |
|---|---|
| Wirkstoff aus Tabelle 1 | 40 % |
| Äthylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37-%-ige wässrige Formaldehyd-Lösung | 0,2 % |
| Silikonöl in Form einer 75-%-igen wässrigen Emuslsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Formulierungsbeispiele für Wirkstoffgemische (flüssig) (% = Gewichtsprozent)

| 16. Emulsions-Konzentrate | a) | b) | c) |
|---|---|---|---|
| Wirkstoffgemisch: Antidot aus Tabelle 1 und eines der Herbizide H-1 bis H-61 im Verhältnis 1 : 1 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 17. Emulsions-Konzentrate | a) | b) | c) |
|---|---|---|---|
| Wirkstoffgemisch: Antidot aus Tabelle 1 und eines der Herbizide H-1 bis H-61 im Verhältnis 1 : 3 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 18. Emulsions-Konzentrate | a) | b) | c) |
|---|---|---|---|
| Wirkstoffgemisch: Antidot aus Tabelle 1 und eines der Herbizide H-1 bis H-61 im Verhältnis 2 : 1 | 25 % | 40 % | 50 % |
| Ca-Dodecylbenzolsulfonat | 5 % | 8 % | 6 % |
| Ricinusöl-polyäthylenglykoläther(36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

19. Emulsions-Konzentrate

| | a) | b) | c) |
|---|---|---|---|
| Wirkstoffgemisch: Antidot aus Tabelle 1 und N-(1-Isopropyl-2-methyl-1-propen-1-yl)-N-(2-methoxyäthyl)-chloracetamid im Verhältnis 1 : 1 | 25 % | 40 % | 50 % |
| Ca-Dodecylbenzolsulfonat | 5 % | 8 % | 6 % |
| Ricinusöl-polyäthylenglykoläther (30 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

20. Emulsions-Konzentrate

| | a) | b) | c) |
|---|---|---|---|
| Wirkstoffgemisch: Antidot aus Tabelle 1 und N-(1-Isopropyl-2-methyl-1-propen-1-yl)-N-(2-methoxyäthyl)-chloracetamid im Verhältnis 1 : 3 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

21. Lösungen

| | a) | b) | c) | d) |
|---|---|---|---|---|
| Wirkstoffgemisch: Antidot aus Tabelle 1 und eines der Herbizide H-1 bis H-61 im Verhältnis 1 : 4 | 80 % | 10 % | 5 % | 95 % |
| Äthylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160 - 190° C) | - | - | 94 % | - |

Die Lösungen sind zur Anwendung in Form kleinster Tropfen geeignet.

22. Lösungen

| | a) | b) | c) | d) |
|---|---|---|---|---|
| Wirkstoffgemisch: Antidot aus Tabelle 1 und eines der Herbizide H-1 bis H-61 im Verhältnis 5 : 2 | 80 % | 10 % | 5 % | 95 % |
| Äthylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160 - 190° C) | - | - | 94 % | - |

Die Lösungen sind zur Anwendung in Form kleinster Tropfen geeignet.

23. Lösungen

| | a) | b) | c) | d) |
|---|---|---|---|---|
| Wirkstoffgemisch: Antidot aus Tabelle 1 und eines der Herbizide H-1 bis H-61 im Verhältnis 1 : 1 | 80 % | 10 % | 5 % | 95 % |
| Äthylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160 - 190° C) | - | - | 94 % | - |

Die Lösungen sind zur Anwendung in Form kleinster Tropfen geeignet.

| 24. Lösungen | a) | b) | c) | d) |
|---|---|---|---|---|
| Wirkstoffgemisch: Antidot aus Tabelle 1 und N-(1-Isopropyl-2-methyl-1-propen-1-yl)-N-(2-methoxyäthyl)-chloracetamid im Verhältnis 1 : 1 | 80 % | 10 % | 5 % | 95 % |
| Äthylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160 - 190° C) | - | - | 94 % | - |

Die Lösungen sind zur Anwendung in Form kleinster Tropfen geeignet.

| 25. Lösungen | a) | b) | c) | d) |
|---|---|---|---|---|
| Wirkstoffgemisch: Antidote aus Tabelle 1 und N-(1-Isopropyl-2-methyl-1-propen-1-yl)-N-(2-methoxyäthyl)-chloracetamid im Verhältnis 1 : 4 | 80 % | 10 % | 5 % | 95 % |
| Äthylenglykol-monomethyl-äther | 20 % | - | - | - |
| Polyäthylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160 - 190° C) | - | - | 94 % | - |

Die Lösungen sind zur Anwendung in Form kleinster Tropfen geeignet.

| 26. Granulate | a) | b) |
|---|---|---|
| Wirkstoffgemisch: Antidot aus Tabelle 1 und eines der Herbizide H-1 bis H-61 im Verhältnis 1 : 1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 27. Granulate | a) | b) |
|---|---|---|
| Wirkstoffgemisch: Antidot aus Tabelle 1 und N-(1-Isopropyl-2-methyl-1-propen-1-yl)-N-(2-methoxyäthyl)-chloracetamid im Verhältnis 1 : 1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 28. Stäubemittel | a) | b) |
|---|---|---|
| Wirkstoffgemisch: Antidot aus Tabelle 1 und eines der Herbizide H-1 bis H-61 im Verhältnis 1 : 1 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.
Formulierungsbeispiele für Wirkstoffgemische (fest) (% = Gewichtsprozent)

| 29. Spritzpulver | a) | b) | c) |
|---|---|---|---|
| Wirkstoffgemisch: Antidot aus Tabelle 1 und eines der Herbizide H-1 bis H-61 im Verhältnis 1 : 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7 - 8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 30. Spritzpulver | a) | b) | c) |
|---|---|---|---|
| Wirkstoffgemisch: Antidot aus Tabelle 1 und eines der Herbizide H-1 bis H-61 im Verhältnis 1 : 4 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7 - 8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 31. Spritzpulver | a) | b) | c) |
|---|---|---|---|
| Wirkstoffgemisch: Antidot aus Tabelle 1 und eines der Herbizide H-1 bis H-61 im Verhältnis 3 : 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7 - 8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vernischt und in einer geeigneten Mühle gut vermahlen. Man erhält Spritzpulver, die sich mit Wasser zu Suspensionen jeder gewünschten Konzentration verdünnen lassen.

| 32. Emulsions-Konzentrate | |
|---|---|
| Wirkstoffgemisch: Antidot aus Tabelle 1 und eines der Herbizide H-1 bis H-61 im Verhältnis 1 : 1 | 10 % |
| Octylphenolpolyäthylenglykoläther (4 - 5 Mol AeO) | 3 % |
| Ca-Dodecylbenzolsulfonat | 3 % |
| Ricinusölpolyglykoläther (35 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

33. Emulsions-Konzentrate

| | |
|---|---|
| Wirkstoffgemisch: Antidot aus Tabelle 1 und eines der Herbizide H-1 bis H-61 im Verhältnis 5 : 2 | 10 % |
| Octylphenolpolyäthylenglykoläther (4 - 5 Mol AeO) | 3 % |
| Ca-Dodecylbenzolsulfonat | 3 % |
| Ricinusölpolyglykoläther (35 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

34. Emulsions-Konzentrate

| | |
|---|---|
| Wirkstoffgemisch: Antidot aus Tabelle 1 und eines der Herbizide H-1 bis H-61 im Verhältnis 1 : 4 | 10 % |
| Octylphenolpolyäthylenglykoläther (4 - 5 Mol AeO) | 3 % |
| Ca-Dodecylbenzolsulfonat | 3 % |
| Ricinusölpolyglykoläther (35 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 35. Stäubemittel | a) | b) |
|---|---|---|
| Wirkstoffgemisch: Antidot aus Tabelle 1 und eines der Herbizide H-1 bis H-61 im Verhältnis 1 : 1 | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

36. Extruder Granulate

| | |
|---|---|
| Wirkstoffgemisch: Antidot aus Tabelle 1 und eines der Herbizide H-1 bis H-61 im Verhältnis 1 : 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

37. Umhüllungs-Granulate

| | |
|---|---|
| Wirkstoffgemisch: Antidot aus Tabelle 1 und eines der Herbizide H-1 bis H-61 im Verhältnis 1 : 1 | 3 % |
| Polyäthylenglykol (M G 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

38. Suspensions-Konzentrate

| | |
|---|---|
| Wirkstoffgemisch: Antidot aus Tabelle 1 und eines der Herbizide H-1 bis H-61 im Verhältnis 1 : 1 | 40 % |
| Äthylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37-%-ige wässrige Formaldehyd-Lösung | 0,2 % |
| Silikonöl in Form einer 75-%-igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

39. Suspension-Konzentrate

| | |
|---|---|
| Wirkstoffgemisch: Antidot aus Tabelle 1 und eines der Herbizide H-1 bis H-61 im Verhältnis 1 : 4 | 40 % |
| Äthylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37-%-ige wässrige Formaldehyd-Lösung | 0,2 % |
| Silikonöl in Form einer 75-%-igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

40. Suspensions-Konzentrate

| | |
|---|---|
| Wirkstoffgemisch: Antidot aus Tabelle 1 und eines der Herbizide H-1 bis H-61 im Verhältnis 1 : 1 | 40 % |
| Äthylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37-%-ige wässrige Formaldehyd-Lösung | 0,2 % |
| Silikonöl in Form einer 75-%-igen wässrigen Emulsion | 0,8 % |
| Wasser | 32 % |

Der fein gemahlene Wirkstoff wird mit den Zusatzstoffen innig vermsicht. Man erhält so ein Suspensions-Konzentrat, aus welchem durch Verdünnen mit Wasser Suspensionen jeder gewünschten Konzentration hergestellt werden können.

**Biologische Beispiele**

Beispiel 41: Tankmischung im Vorauflaufverfahren bei Mais

Plastikbehälter (Länge x Breite x Höhe = 25 x 17 x 12 cm) werden mit sandiger Lehmerde gefüllt, in die Maissamen der Sorte LG 5 eingesät werden. Nach dem Bedecken der Samen mit Erde wird 2-(3-Chlor-n-propyl)-4-methyl-6-(trichlormethyl)-1,3,5-triazin als Antidot zusammen mit dem Herbizid 2-Chlor-2',6'-dimethyl-N-(2-methoxy-1-methyläthyl)-acetanilid in verdünnter Lösung als Tankmischung auf die Bodenoberfläche gesprüht. 21 Tage nach der Applikation wird die Schutzwirkung des Antidots in Prozent bonitiert. Als Referenz dienen dabei die mit dem Herbizid allein behandelten Pflanzen sowie die vollständig unbehandelte Kontrolle. Bei Aufwandmengen, welche für das Antidot 1,5 kg/ha und für das Herbizid 6,0 kg/ha entsprechen, wird eine

relative Schutzwirkung von 75 % erreicht.

Beispiel 42: Tankmischung im Vorauflaufverfahren bei Mais

Maissamen der Sorte "LG 5" werden im Gewächshaus in Plastiktöpfe (oberer Durchmesser 11 cm), die 0,5 l sandige Lehmerde enthalten, gesät. Nach dem Bedecken der Samen mit Erde wird die als Safener zu prüfende Substanz zusammen mit dem Herbizid N-Chloracetyl-N-(2-methoxy-1-methyläthyl)-2,6-dimethylanilin als Tankmischung in einer Menge, welche 550 l/ha und einer Herbizidmenge von 4 kg und 6 kg/ha entspricht, im Vorauflaufverfahren auf die Bodenoberfläche appliziert. 21 Tage nach der Applikation wird die Schutzwirkung des Safeners in Prozent bonitiert. Als Referenz dient dabei mit dem Herbizid allein behandelter Mais sowie die vollständig unbehandelte Kontrolle. Die Ergebnisse sind in den Tabellen 2 und 3 dargestellt.

**Tabelle 2**

Herbizid: H-44, 4 kg AS/ha

| Safener Nr. | Relative Schutzwirkung in % bei einer Aufwandmenge an Safener von 2 kg/ha | 1 kg/ha | 0,5 kg/ha | 0,25 kg/ha |
|---|---|---|---|---|
| 1 | 50 | 50 | 50 | 0 |
| 7 | 50 | 63 | 50 | 50 |
| 16 | 13 | 13 | 25 | 38 |
| 29 | 63 | 75 | 63 | 63 |
| 30 | 63 | 50 | 50 | 50 |

**Tabelle 3**

Herbizid: H-44, 6 kg AS/ha

| Safener Nr. | Relative Schutzwirkung in % bei einer Aufwandmenge an Safener von 3 kg/ha | 1,5 kg/ha | 0,75 kg/ha | 0,375 kg/ha |
|---|---|---|---|---|
| 1 | 50 | 50 | 63 | 13 |
| 7 | 63 | 63 | 63 | 50 |
| 16 | 13 | 13 | 13 | 0 |
| 29 | 63 | 75 | 75 | 75 |
| 30 | 38 | 38 | 50 | 63 |

Beispiel 43: Tankmischung im Vorauflaufverfahren bei Mais

Plastikbehälter (Länge x Breite x Höhe = 25 x 17 x 12 cm) werden mit sandiger Lehmerde gefüllt und in diese die Maissamen eingesät. Nach dem Bedecken der Samen mit Erde wird die als Safener zu prüfende Substanz zusammen mit dem Herbizid N-Chloracetyl-N-(2-methoxy-1-methyläthyl)-2,6-dimethylanilin in verdünnter Lösung als Tankmischung auf die Bodenoberfläche gesprüht, wobei die Menge an Herbizid 4 kg und 6 kg/ha entspricht. 21 Tage nach der Applikation wird die Schutzwirkung des Safeners in Prozent bonitiert. Als Referenz dienen dabei die mit dem Herbizid allein behandelten Pflanzen sowie die vollständig unbehandelte Kontrolle. Die Ergebnisse zeigen die nachfolgenden Tabellen 4 und 5.

**Tabelle 4**

Herbizid: H-44, 4 kg AS/ha

| Safener Nr. | Relative Schutzwirkung in % bei einer Aufwandmenge an Safener in kg AS/ha von 2 | 1 | 0,5 | 0,333 | 0,25 |
|---|---|---|---|---|---|
| 1 | 63 | 63 | 50 | 13 | 25 |
| 7 | 63 | 63 | 50 | 38 | 13 |

**Tabelle 5**

Herbizid: H-44, 6 kg AS/ha

| Safener Nr. | Relative Schutzwirkung in % bei einer Aufwandmenge an Safener in kg AS/ha von 3 | 1,5 | 0,75 | 0,5 | 0,375 |
|---|---|---|---|---|---|
| 1 | 75 | 50 | 38 | 25 | 25 |
| 7 | 75 | 63 | 63 | 38 | 38 |

Beispiel 44: Einzelapplikation von Safener und Herbizid im Vorauflaufverfahren bei Mais

Plastiktöpfe (oberer Durchmesser 9 cm, Inhalt 315 ml) werden mit sandig-toniger Lehmerde gefüllt. Maissamen der Sorte "LG 5" werden eingesät, mit Erde bedeckt und angegossen. Die als Safener zu prüfende Substanz wird in Wasser gelöst und in einer Menge, welche 550 l/ha und einer Safenermenge von 1,5 kg AS/ha entspricht, auf die Bodenoberfläche aufgesprüht. Anschliessend wird das Herbizid N-Chloracetyl-N-(2-methoxy-1-methyläthyl)-2,6-dimethylanilin in Wasser gelöst und in gleicher Weise appliziert, wobei die Herbizidmenge 6 kg AS/ha entspricht. Die Testpflanzen werden im Gewächshaus unter folgenden Bedingungen kultiviert: 13 Stunden Licht (24 - 26°C), 11 Stunden Dunkelheit (20 - 22°C) und ca. 40 - 60 % relativer Luftfeuchtigkeit. Zwei Wochen nach der Applikation wird die Schutzwirkung des Safeners in Prozent bonitiert. Als Referenz dienen dabei die mit dem Herbizid allein behandelten Pflanzen sowie die vollständig unbehandelten Kontrollen. Die Ergebnisse sind in Tabelle 6 dargestellt.

**Tabelle 6**

| Safener Nr. | Relative Schutzwirkung in % |
|---|---|
| 27 | 13 |
| 18 | 50 |
| 4 | 50 |
| 8 | 13 |
| 21 | 13 |
| 1 | 25 |
| 9 | 13 |
| 22 | 25 |
| 28 | 38 |
| 14 | 13 |
| 7 | 38 |
| 23 | 50 |
| 16 | 75 |
| 29 | 63 |
| 30 | 63 |
| 31 | 63 |
| 32 | 38 |

Beispiel 45: Einzelapplikation von Safener und Herbizid im Vorauflaufverfahren bei Sorghum

Plastiktöpfe (oberer Durchmesser 6,5 cm, Inhalt 165 ml) werden mit sandig-toniger Lehmerde gefüllt. Sorghumsamen der Sorte Funk G-623 werden eingesät, mit Erde bedeckt und angegossen. Die als Safener zu prüfende Substanz wird in Wasser gelöst und in einer Menge, welche 550 l/ha und einer Safenermenge von 1,5 kg AS/ha entspricht, auf die Bodenoberfläche aufgesprüht. Anschliessend wird das Herbizid N-Chloracetyl-N-(2-methoxy-1-methyläthyl)-2-äthyl-6-methylanilin in Wasser gelöst und in gleicher Weise appliziert, wobei die Herbizidmenge 1,5 kg AS/ha entspricht. Die Testpflanzen werden im Gewächshaus unter folgenden Bedingungen kultiviert: 13 Stunden Licht (24 - 26°C), 11 Stunden Dunkelheit (20 - 22°C) und ca. 40 - 60 % relativer Luftfeuchtigkeit. Zwei Wochen nach der Applikation wird die Schutzwirkung des Safeners in Prozent bonitiert. Als Referenz dienen dabei die mit dem Herbizid allein behandelten Pflanzen sowie die vollständig unbehandelten Kontrollen. Die Ergebnisse zeigt Tabelle 7.

**Tabelle 7**

| Safener Nr. | Relative Schutzwirkung in % |
|---|---|
| 8 | 50 |
| 31 | 38 |

**Patentansprüche**

1. Verfahren zum Schützen von Mais- und Sorghumpflanzen gegen schädigende Wirkungen von herbiziden Chloracetaniliden der Formel II

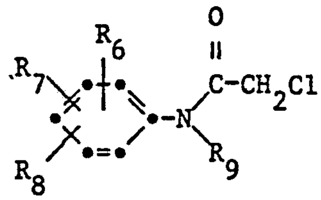

(II)

worin
$R_6$ Wasserstoff, $C_1$-$C_4$-Alkyl, Methoxy oder Chlor,
$R_7$ Wasserstoff oder $C_1$-$C_3$-Alkyl und
$R_8$ Wasserstoff, Methyl oder Äthyl
bedeuten, mit der Massgabe, dass $R_6$, $R_7$ und $R_8$ gemeinsam nicht mehr als 6 Kohlenstoffatome aufweisen, und
$R_9$ für Äthyl, Isopropyl, 1-methyl-2-methoxyäthyl, $-CH_2-R_{10}$, $-CH(CH_3)-R_{11}$, $-CH_2-CH(R_{12})-R_{13}$, $-CH(CH_3)-CH(R_{14})-R_{15}$, $C_3$-$C_4$-Alkinyl oder 5-Methyl-1,3,4-oxadiazol-2-yl steht, worin $R_{10}$ $C_1$-$C_4$-Alkoxy, $C_1$-$C_3$-Alkoxycarbonyl, Dimethylaminocarbonyl, 2-Propinylaminocarbonyl, Benzoyl, 4-Chlorbenzoyl, Cyan, 2-Furanyl, 2-Tetrahydrofuranyl, 1,3-Dioxolan-2-yl, 1,3-Dioxan-2-yl, 1H-Pyrazol-1-yl, 3,5-Dimethyl-1H-pyrazol-1-yl, 1H-1,2,4-Triazol-1-yl, 1-Methyl-5-methylthio-1,3,4-triazol-2-yl oder $-P(O)(OR_{16})(OR_{17})$, worin $R_{16}$ und $R_{17}$ unabhängig voneinander für Methyl oder Äthyl stehen, $R_{11}$ Methoxycarbonyl oder 1,3-Dioxolan-2-yl, $R_{12}$ Wasserstoff und $R_{13}$ $C_1$-$C_4$-Alkoxy oder Allyloxy oder $R_{12}$ Methyl und $R_{13}$ $C_1$-$C_2$-Alkoxy oder $R_{12}$ und $R_{13}$ Äthoxy und $R_{14}$ Wasserstoff und $R_{15}$ $C_1$-$C_2$-Alkoxy oder $R_{14}$ Methyl und $R_{15}$ Methoxy bedeuten dadurch gekennzeichnet, dass man diese Pflanzen, Teile dieser Pflanzen oder die Anbauflächen für diese Pflanzen gleichzeitig mit einem Herbizid der Formel II oder vor oder nach der Anwendung eines Herbizids der Formel II mit einer antagonistisch wirksamen Menge einer Verbindung der Formel I

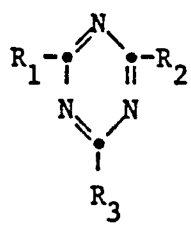

(I)

worin
$R_1$, $R_2$ und $R_3$ unabhängig voneinander $C_1$-$C_5$-Alkyl, welches unsubstituiert oder durch $C_3$-$C_6$-Cycloalkyl monosubstituiert oder durch Halogen ein- oder mehrfach substituiert ist, $C_3$-$C_6$-Cycloalkyl, welches unsubstituiert oder ein- oder mehrfach durch Halogen substituiert ist, bedeuten, oder einer der Substituenten $R_1$, $R_2$ und $R_3$ auch für Halogen oder $-NR_4R_5$ steht, worin $R_4$ Wasserstoff oder $C_1$-$C_5$-Alkyl und $R_5$ Wasserstoff, $C_1$-$C_5$-Alkyl, Monochloracetyl oder Dichloracetyl bedeuten, oder einem Mittel, welche eine dieser Verbindungen enthält, behandelt.

2. Verfahren nach Anspruch 1, dadurch gekennzeichnet, dass man eine Verbindung der Formel I, in welcher zwei der Substituenten $R_1$, $R_2$ und $R_3$ unabhängig voneinander $C_1$-$C_5$-Alkyl, welches unsubstituiert oder durch $C_3$-$C_6$-Cycloalkyl monosubstituiert oder durch Halogen ein- oder mehrfach substituiert ist, $C_3$-$C_6$-Cycloalkyl, welches unsubstituiert oder ein- oder mehrfach durch Halogen substituiert ist, bedeuten, oder einer der Substituenten auch für Halogen steht, und der dritte Substituent für $C_3$-$C_6$-Cycloalkyl steht, verwendet.

3. Verfahren nach Anspruch 2, dadurch gekennzeichnet, dass man eine Verbindung der Formel I, in welcher zwei der Substituenten $R_1$, $R_2$ und $R_3$ unabhängig voneinander $C_1$-$C_5$-Alkyl, welches unsubstituiert oder durch Cyclopropyl monosubstituiert oder durch Halogen ein- oder mehrfach substituiert ist, oder Cyclopropyl bedeuten, oder einer der Substituenten auch für Halogen steht, und der dritte Substituent für Cyclopropyl steht, verwendet.

4. Herbizides Mittel, dadurch gekennzeichnet, dass es neben einer herbizid wirksamen Menge eines Chloracetanilids der Fromel II

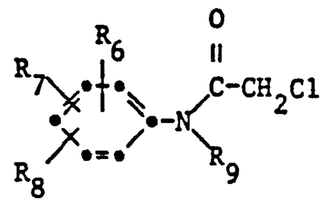

(II)

worin
$R_6$ Wasserstoff, $C_1$-$C_4$-Alkyl, Methoxy oder Chlor,
$R_7$ Wasserstoff oder $C_1$-$C_3$-Alkyl und
$R_8$ Wasserstoff, Methyl oder Äthyl
bedeuten, mit der Massgabe, dass $R_6$, $R_7$ und $R_8$ gemeinsam nicht mehr als 6 Kohlenstoffatome aufweisen, und
$R_9$ für Äthyl, Isopropyl 1-Methyl-2-methoxyäthyl, $-CH_2-R_{10}$, $-CH(CH_3)-R_{11}$, $-CH_2-CH(R_{12})-R_{13}$, $-CH(CH_3)-CH(R_{14})-R_{15}$, $C_3$-$C_4$-Alkinyl oder 5-Methyl-1,3,4-oxadiazol-2-yl steht, worin $R_{10}$ $C_1$-$C_4$-Alkoxy, $C_1$-$C_3$-Alkoxycarbonyl, Dimethylaminocarbonyl, 2-Propinylaminocarbonyl, Benzoyl, 4-Chorbenzoyl, Cyan, 2-Furanyl, 2-Tetrahydrofuranyl, 1,3-Dioxolan-2-yl, 1,3-Dioxan-2-yl, 1H-Pyrazol-1-yl, 3,5-Dimethyl-1H-pyrazol-1-yl, 1H-1,2,4-Triazol-1-yl, 1-Methyl-5-methylthio-1,3,4-triazol-2-yl oder $-P(O)(OR_{16})(OR_{17})$, worin $R_{16}$ und $R_{17}$ unabhängig voneinander für Methyl oder Äthyl stehen, $R_{11}$ Methoxycarbonyl oder 1,3-Dioxolan-2-yl, $R_{12}$ Wasserstoff und $R_{13}$ $C_1$-$C_4$-Alkoxy oder Allyloxy oder $R_{12}$ Methyl und $R_{13}$ $C_1$-$C_2$-Alkoxy oder $R_{12}$ und $R_{13}$ Äthoxy und $R_{14}$ Wasserstoff und $R_{15}$ $C_1$-$C_2$-Alkoxy oder $R_{14}$ Methyl und $R_{15}$ Methoxy bedeuten, eine die schädigende Wirkung des Herbizids II antagonisierende Menge einer Verbindung der Formel I

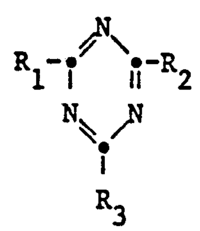

(I),

worin
$R_1$, $R_2$ und $R_3$ unabhängig voneinander $C_1$-$C_5$-Alkyl, welches unsubstituiert oder durch $C_3$-$C_6$-Cycloalkyl monosubstituiert oder durch Halogen ein- oder mehrfach substituiert ist, $C_3$-$C_6$-Cycloalkyl, welches unsubstituiert oder ein- oder mehrfach durch Halogen substituiert ist, bedeuten, oder einer der Substituenten $R_1$, $R_2$ und $R_3$ auch für Halogen oder $-NR_4R_5$ steht, worin $R_4$ Wasserstoff oder $C_1$-$C_5$-Alkyl und $R_5$ Wasserstoff, $C_1$-$C_5$-Alkyl, Monochloracetyl oder Dichloracetyl bedeuten, enthält.

5. Mittel nach Anspruch 4, dadurch gekennzeichnet, dass in der Verbindung der Formel I zwei der Substituenten $R_1$, $R_2$ und $R_3$ unabhängig voneinander $C_1$-$C_5$-Alkyl, welches unsubstituiert oder durch $C_3$-$C_6$-Cycloalkyl monosubstituiert oder durch Halogen ein- oder mehrfach substituiert ist, $C_3$-$C_6$-Cycloalkyl, welches unsubstituiert oder ein- oder mehrfach durch Halogen substituiert ist, bedeuten, oder einer der Substituenten auch für Halogen steht, und der dritte Substituent $C_3$-$C_6$-Cycloalkyl bedeutet.

6. Mittel nach Anspruch 5, dadurch gekennzeichnet, dass in der Verbindung der Formel I zwei der Substituenten $R_1$, $R_2$ und $R_3$ unabhängig voneinander $C_1$-$C_5$-Alkyl, welches unsubstituiert oder durch Cyclopropyl monosubstituiert oder durch Halogen ein- oder mehrfach substituiert ist, oder Cyclopropyl bedeuten, oder einer der Substituenten auch für Halogen steht, und der dritte Substituent Cyclopropyl bedeutet.

**Claims**

1. A process for the protection of maize and sorghum plants against the harmful effects of herbicidal chloroacetanilides of the formula II

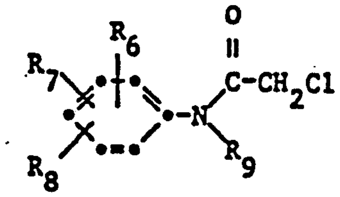

(II)

wherein
$R_6$ is hydrogen, $C_1$-$C_4$-alkyl, methoxy or chlorine,
$R_7$ is hydrogen or $C_1$-$C_3$-alkyl, and
$R_8$ is hydrogen, methyl or ethyl,
with the proviso that $R_6$, $R_7$ and $R_8$ together do not contain more than 6 carbon atoms, and
$R_9$ is ethyl, isopropyl, 1-methyl-2-methoxyethyl, $-CH_2-R_{10}$, $-CH(CH_3)-R_{11}$, $-CH_2-CH(R_{12})-R_{13}$, $-CH(CH_3)-CH(R_{14})-R_{15}$, $C_3$-$C_4$-alkynyl or 5-methyl-1,3,4-oxadiazol-2-yl, in which $R_{10}$ is $C_1$-$C_4$-alkoxy, $C_1$-$C_3$-alkoxycarbonyl, dimethylaminocarbonyl, 2-propynylaminocarbonyl, benzoyl, 4-chloro-benzoyl, cyano, 2-furanyl, 2-tetrahydrofuranyl, 1,3-dioxolan-2-yl, 1,3-dioxan-2-yl, 1H-pyrazol-1-yl, 3,5-dimethyl-1H-pyrazol-1-yl, 1H-1,2,4-triazol-1-yl, 1-methyl-5-methylthio-1,3,4-triazol-2-yl or $-P(O)(OR_{16})(OR_{17})$, in which $R_{16}$ and $R_{17}$ independently of one another are each methyl or ethyl, $H_{11}$ is methoxycarbonyl or 1,3-dioxolan-2-yl, $R_{12}$ is hydrogen, and $R_{13}$ is $C_1$-$C_4$-alkoxy or allyloxy, or $R_{12}$ is methyl and $R_{13}$ is $C_1$-$C_2$-alkoxy, or $R_{12}$ and $R_{13}$ are ethoxy and $R_{14}$ is hydrogen and $R_{15}$ is $C_1$-$C_2$-alkoxy, or $R_{14}$ is methyl and $R_{15}$ is methoxy, which process comprises treating these plants, parts of these plants or cultivated areas for these plants, at the same time as with a herbicide of the formula II or before or after the application of a herbicide of the formula II, with an antagonistically effective amount of a compound of the formula I

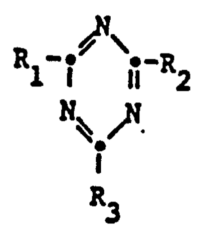

(I)

wherein
$R_1$, $R_2$ and $R_3$ independently of one another are each $C_1$-$C_5$-alkyl which is unsubstituted or monosubstituted by $C_3$-$C_6$-cycloalkyl or mono- or polysubstituted by halogen, or are $C_3$-$C_6$-cycloalkyl which is unsubstituted or mono- or polysubstituted by halogen, or one of the substituents $R_1$, $R_2$ and $R_3$ is also halogen or $-NH_4R_5$, in which $R_4$ is hydrogen or $C_1$-$C_5$-alkyl and $R_5$ is hydrogen, $C_1$-$C_5$-alkyl, monochloroacetyl or dichloroacetyl, or with a composition containing one of these compounds.

2. A process according to claim 1, which process comprises applying a compound of the formula I in which two of the substituents $R_1$, $R_2$ and $R_3$ independently of one another are each $C_1$-$C_5$-alkyl which is unsubstituted or monosubstituted by $C_3$-$C_6$-cycloalkyl or mono- or polysubstituted by halogen, or are $C_3$-$C_6$-cycloalkyl which is unsubstituted or mono- or polysubstituted by halogen, or one of the substituents is also halogen, and the third substituent is $C_3$-$C_6$-cycloalkyl.

3. A process according to claim 2, which process comprises applying a compound of the formula I in which two of the substituents $R_1$, $R_2$ and $R_3$ independently of one another are each $C_1$-$C_5$-alkyl which is unsubstituted or monosubstituted by cyclopropyl or mono- or polysubstituted by halogen, or are cyclopropyl, or one of the substituents is also halogen, and the third substituent is cyclopropyl.

4. A herbicidal composition, which composition contains, in addition to a herbicidally active amount of a chloroacetanilide of the formula II

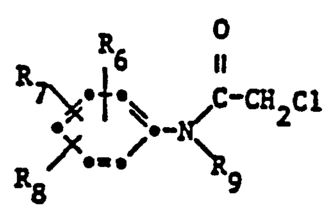

(II)

wherein
$R_6$ is hydrogen $C_1$-$C_4$-alkyl, methoxy or chlorine,

$R_7$ is hydrogen or $C_1$-$C_3$-alkyl, and
$R_8$ is hydrogen, methyl or ethyl,
with the proviso that $R_6$, $R_7$ and $R_8$ together do not contain more than 6 carbon atoms, and
$R_9$ is ethyl, isopropyl, 1-methyl-2-methoxyethyl, $-CH_2-R_{10}$, $-CH(CH_3)-R_{11}$, $-CH_2-CH(R_{12})-R_{13}$, $-CH(CH_3)-CH(R_{14})-R_{15}$, $C_3$-$C_4$-alkynyl or 5-methyl-1,3,4-oxadiazol-2-yl, in which $R_{10}$ is $C_1$-$C_4$-alkoxy, $C_1$-$C_3$-alkoxycarbonyl, dimethylaminocarbonyl, 2-propynylaminocarbonyl, benzoyl, 4-chlorobenzoyl, cyano, 2-furanyl, 2-tetrahydrofuranyl, 1,3-dioxolan-2-yl, 1,3-dioxan-2-yl, 1H-pyrazol-1-yl, 3,5-dimethyl-1H-pyrazol-1-yl, 1H-1,2,4-triazol-1-yl, 1-methyl-5-methylthio-1,3,4-triazol-2-yl or $-P(O)(OR_{16})(OR_{17})$, in which $R_{18}$ and $H_{17}$ independently of one another are each methyl or ethyl, $R_{11}$ is methoxycarbonyl or 1,3-dioxolan-2-yl, $R_{12}$ is hydrogen, and $R_{13}$ is $C_1$-$C_4$-alkoxy or allyloxy, or $R_{12}$ is methyl and $R_{13}$ is $C_1$-$C_2$-alkoxy, or $R_{12}$ and $R_{13}$ are ethoxy and $R_{14}$ is hydrogen and $R_{15}$ is $C_1$-$C_2$-alkoxy, or $R_{14}$ is methyl and $R_{15}$ is methoxy, an amount of a compound of formula I

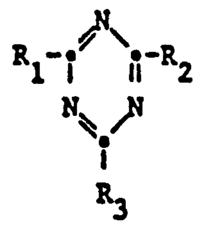

(I)

sufficient to antagonise the harmful effect of herbicide II, in which compound of the formula I $R_1$, $R_2$ and $R_3$ independently of one another are each $C_1$-$C_5$-alkyl which is unsubstituted or monosubstituted by $C_3$-$C_6$-cycloalkyl or mono- or polysubstituted by halogen, or are $C_3$-$C_6$-cycloalkyl which is unsubstituted or mono- or polysubstituted by halogen, or one of the substituents $R_1$, $R_2$ and $R_3$ is also halogen or $-NR_4R_5$, in which $R_4$ is hydrogen or $C_1$-$C_5$-alkyl and $R_5$ is hydrogen, $C_1$-$C_5$-alkyl, monochloroacetyl or dichloroacetyl.

5. A composition according to claim 4, wherein in the compound of the formula I two of the substituents $R_1$, $R_2$ and $R_3$ independently of one another are each $C_1$-$C_5$-alkyl which is unsubstituted or monosubstituted by $C_3$-$C_6$-cycloalkyl or mono- or polysubstituted by halogen, or are $C_3$-$C_6$-cycloalkyl which is unsubstituted or mono- or polysubstituted by halogen, or one of the substituents is also halogen, and the third substituent is $C_3$-$C_6$-cycloalkyl.

6. A composition according to claim 5, wherein in the compound of the formula I two of the substituents $R_1$, $R_2$ and $R_3$ independently of one another are each $C_1$-$C_5$-alkyl which is unsubstituted or monosubstituted by cyclopropyl or mono- or polysubstituted by halogen, or are cyclopropyl, or one of the substituents is also halogen, and the third substituent is cyclopropyl.

**Revendications**

1. Procédé de protection de plantes du genre mais et sorgho vis-à-vis des actions nocives de chloroacétanilides herbicides de formule II

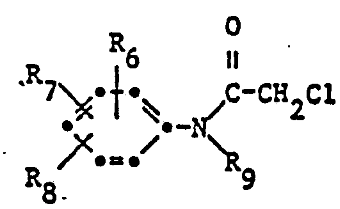

(II)

dans laquelle
$R_6$ représente l'hydrogène un, alkyle $C_1$-$C_4$, le méthoxy ou le chlore,
$R_7$ l'hydrogène ou un alkyle $C_1$-$C_3$ et
$R_8$ l'hydrogène, le méthyle ou l'éthyle avec la condition que $R_6$, $R_7$ et $R_8$ ne présentent ensemble pas plus de 6 atomes de carbone, et
$R_9$ représente l'éthyle, l'isopropyle, le 1-méthyl-2-méthoxyéthyle, $-CH_2-R_{10}$, $-CH(CH_3)-R_{11}$, $-CH_2-CH(R_{12})-R_{13}$, $-CH(CH_3)-CH(R_{11})-R_{15}$, $C_3$-$C_4$ alcynyle ou 5-méthyl-1,3,4-oxadiazolyl-2, dans lesquels $R_{10}$ représente un alcoxy $C_1$-$C_4$, alcoxycarbonyle $C_1$-$C_3$, diméthylaminocarbonyle, 2-propynylaminocarbonyle, benzoyle, 4-chlorobenzoyle, cyano, 2-furannyle, 2-tétrahydrofurannyle, 1,3-dioxolanyl-2,1,3-dioxanyl-2, 1H-pyrazolyl-1, 3,5 diméthyl-1H-pyrazolyl-1, 1H-1,2,4-triazolyl-1, 1-méthyl-5-méthylthio-1,3,4-triazolyl-2 ou $-P(O)(OR_{16})(OR_{17})$, dans lesquels $R_{16}$ et $R_{17}$ représentent indépendamment l'un de l'autre le méthyle ou l'éthyle, $R_{11}$ représente le méthoxycarbonyle ou le 1,3-dioxolanyl-2, $R_{12}$ l'hydrogène et $R_{13}$ un alcoxy $C_1$-$C_4$ ou l'allyloxy ou $R_{12}$ représente le méthyle et $R_{13}$ un alcoxy-$C_1$-$C_2$ ou $R_{12}$ et $R_{13}$ représentent l'éthoxy et $R_{14}$ l'hydrogène et $R_{15}$ un alcoxy-$C_1$-$C_2$

ou $R_{14}$ représente le méthyle et $R_{15}$ le méthoxy caractérisé en ce qu'on traite ces plantes, des parties de ces plantes ou les surfaces cultivées de ces plantes simultanément avec un herbicide de formule II ou avant ou après l'utilisation d'un herbicide de formule II avec une quantité efficace neutralisante d'un composé de formule I.

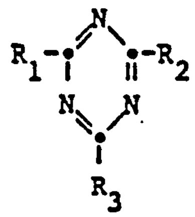

(I)

dans laquelle

$R_1$, $R_2$ et $R_3$ représentent indépendamment l'un de l'autre un alkyle $C_1$-$C_5$, qui est non substitué ou monosubstitué par un cycloalkyle $C_3$-$C_6$ ou est mono- ou polysubstitué par un halogène, un cycloalkyle $C_3$-$C_6$ qui est non substitué ou est mono- ou polysubstitué par un halogène, ou l'un des substituants $R_1$, $R_2$ et $R_3$ représente aussi un halogène ou $-NR_4R_5$, dans lequel $R_4$ représente l'hydrogène ou un alkyle $C_1$-$C_5$ et $R_5$ l'hydrogène, un alkyle $C_1$-$C_5$, le monochloroacétyle ou le dichloroacétyle, ou avec un moyen qui contient un de ces composés.

2. Procédé selon la revendication 1, caractérisé en ce qu'on utilise un composé de formule I dans laquelle deux des substituants $R_1$, $R_2$ et $R_3$ représentent indépendamment l'un de l'autre un alkyle $C_1$-$C_5$, qui est non substitué ou monosubstitué par un cycloalkyle $C_3$-$C_6$ ou est mono- ou polysubstitué par un halogène, un cycloalkyle $C_3$-$C_6$ qui est non substitué ou est mono- ou polysubstitué par un halogène, ou l'un des substituants représente aussi un halogène, et le troisième substituant représente un cycloalkyle $C_3$-$C_6$.

3. Procédé selon la revendication 2, caractérisé en ce qu'on utilise un composé de formule I dans laquelle deux des substituants $R_1$, $R_2$ et $R_3$ représentent indépendamment l'un de l'autre un alkyle $C_1$-$C_5$, qui est non substitué ou monosubstitué par un cyclopropyle ou est mono- ou polysubstitué par un halogène, un cycloalkyle $C_3$-$C_6$, qui est non substitué ou est mono- ou polysubstitué par un halogène, ou représentent le cyclopropyle, ou l'un des substituants représente aussi un halogène, et le troisième substituant représente le cyclopropyle.

4. Agent herbicide, caractérisé en ce que, il comporte, outre une quantité herbicide efficace d'un chloroacétanilide de formule II

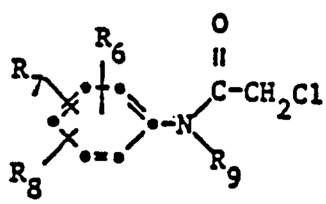

(II)

dans laquelle

$R_6$ représente l'hydrogène, un alkyle $C_1$-$C_4$, le méthoxy ou le chlore,

$R_7$ l'hydrogène ou un alkyle $C_1$-$C_3$ et

$R_8$ l'hydrogène, le méthyle ou l'éthyle

avec la condition que $R_6$, $R_7$ et $R_8$ ne présentent ensemble pas plus de 6 atomes de carbone, et

$R_9$ représente l'éthyle, l'isopropyle, le 1-méthyl-2-méthoxyéthyle, $-CH_2-R_{10}$, $-CH(CH_3)-R_{11}$, $-CH_2-CH(R_{12})-R_{13}$, $-CH(CH_3)-CH(R_{11})-R_{15}$, $C_3$-$C_4$ alcynyle ou 5-méthyl-1,3,4-oxadiazolyl-2, dans lesquels $R_{10}$ représente un alcoxy $C_1$-$C_4$, alcoxycarbonyle $C_1$-$C_3$, diméthylaminocarbonyle, 2-propynylaminocarbonyle, benzoyle, 4-chlorobenzoyle, cyano, 2-furannyle, 2-tétrahydrofurannyle, 1,3-dioxolanyl-2, 1,3-dioxanyl-2, 1H-pyrazolyl-1,3,5-diméthyl-1H-pyrazolyl-1, 1H-1,2,4-triazolyl-1, 1-méthyl-5-méthylthio-1,3,4-triazolyl-2 ou $-P(O)(OR_{16})(OR_{17})$, dans lesquels $R_{16}$ et $R_{17}$ représentent indépendamment l'un de l'autre le méthyle ou l'éthyle, $R_{11}$ représente le méthoxycarbonyle ou le 1,3-dioxolanyl-2, $R_{12}$ l'hydrogène et $R_{13}$ un alcoxy $C_1$-$C_4$ ou l'allyloxy ou $R_{12}$ représente le méthyle et $R_{13}$ un alcoxy-$C_1$-$C_2$ ou $R_{12}$ et $R_{13}$ représentent l'éthoxy et $R_{14}$ l'hydrogène et $R_{15}$ un alcoxy-$C_1$-$C_2$ ou $R_{14}$ représente le méthyle et $R_{15}$ le méthoxy; une quantité neutralisante de l'action nocive de l'herbicide II d'un composé de formule I

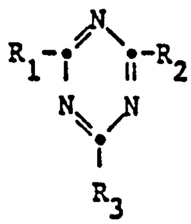

(I)

dans laquelle
$R_1$, $R_2$ et $R_3$ représentent indépendamment l'un de l'autre un alkyle $C_1$-$C_5$, qui est non substitué ou monosubstitué par un cycloalkyle $C_3$-$C_6$ ou est mono- ou polysubstitué par un halogène, un cycloalkyle $C_3$-$C_6$, qui est non substitué ou est mono- ou polysubstitué par un halogène, ou l'un des substituants $R_1$, $R_2$ et $R_3$ représente aussi un halogène ou $-NR_4R_5$, dans lequel $R_4$ représente l'hydrogène ou un alkyle $C_1$-$C_5$ et $R_5$ l'hydrogène, un alkyle $C_1$-$C_5$, le monochloroacétyle ou le dichloroacétyle.

5. Agent selon la revendication 4, caractérisé en ce que, dans le composé de formule I deux des substituants $R_1$, $R_2$ et $R_3$ représentent indépendamment l'un de l'autre un alkyle $C_1$-$C_5$, qui est non substitué ou monosubstitué par un cycloalkyle $C_3$-$C_6$ ou est mono- ou polysubstitué par un halogène, un cycloalkyle $C_3$-$C_6$, qui est non substitué ou est mono- ou polysubstitué par un halogène, ou l'un des substituants, représente aussi un halogène et le troisième substituant représente un cycloalkyle $C_3$-$C_6$.

6. Agent selon la revendication 5, caractérisé en ce que, dans le composé de formule I, deux des substituants $R_1$, $R_2$ et $R_3$ représentent indépendamment l'un de l'autre un alkyle $C_1$-$C_5$, qui est non substitué ou monosubstitué par un cyclopropyle ou est mono- ou polysubstitué par un halogène, ou représentent le cyclopropyle ou l'un des substituant représente aussi un halogène, et le troisième substituant représente le cyclopropyle.